# EUROPÄISCHE PATENTSCHRIFT

(11) **EP 3 586 899 B1**
(45) Veröffentlichungstag und Bekanntmachung des Hinweises auf die Patenterteilung: **22.04.2026**
(21) Anmeldenummer: 18179524.6
(22) Anmeldetag: 25.06.2018
(51) Int. Cl.: A61M 25/00, A61M 25/01

(54) **KATHETER, INSBESONDERE BALLONKATHETER, SOWIE VERFAHREN ZUR HERSTELLUNG DES KATHETERS**
CATHETER, IN PARTICULAR BALLOON CATHETER, AND A METHOD FOR MANUFACTURING THE SAME
CATHÉTER, EN PARTICULIER CATHÉTER À BALLONNET AINSI QUE PROCÉDÉ DE FABRICATION DE CATHÉTER

(43) Veröffentlichungstag der Anmeldung: 01.01.2020
(73) Patentinhaber: BIOTRONIK AG, 8180 Bülach (CH)
(72) Erfinder: Quint, Bodo, 79802 Dettighofen (DE)
(74) Vertreter: WSL Patentanwälte Partnerschaft mbB

(56) Entgegenhaltungen:
- EP-A1- 1 199 082
- EP-A1- 1 787 673
- EP-A1- 2 974 765
- WO-A1-01/45788
- WO-A1-99/47203
- US-A1- 2007 021 771
- US-A1- 2010 217 234

## Beschreibung

Die vorliegende Erfindung betrifft einen Katheter, insbesondere einen Ballonkatheter, sowie ein Verfahren zur Herstellung eines solchen Katheters.

Ein derartiger Katheter stellt eine rohrförmige Struktur dar, die z.B. zu therapeutischen Zwecken in ein Körperlumen, insbesondere Gefäß, eines Patienten einführbar ist. Ein spezielles Anwendungsgebiet stellen PCTA-Katheter dar, die bei der perkutanen transluminalen koronaren Angioplastie (PTCA für Percutaneous Transluminal Coronary Angioplasty) eingesetzt werden, um ein verengtes oder verschlossenes Herzkranzgefäß zu erweitern bzw. wiederzueröffnen. Der Katheter ist hierzu als Ballonkatheter ausgestaltet und weist an einem distalen Ende des Katheters einen mit einem Fluid (insbesondere Druckluft) aufblasbaren Ballon auf, der in der Gefäßverengung expandiert wird. Ein derartiger Katheter kann insbesondere mit Hilfe eines Führungsdrahts zur Gefäßverengung geführt bzw. geschoben werden.

Die vorliegende Erfindung lässt sich jedoch grundsätzlich auch auf andere Katheter anwenden, z.B. Ballonkatheter, die zur Entfaltung von Stents oder Herzklappenprothesen verwendet werden.

Aus dem Stand der Technik sind Katheter bekannt, die einen zweigliedrigen Schaftaufbau aufweisen, bei dem z.B. ein proximales Schaftsegment, das üblicherweise durch ein metallisches rohrförmiges Element gebildet wird (z.B. Hypotube) und an einer Fügestelle mit einem koaxialen Abschnitt ausgestattet ist, wobei über die Fügestelle, an der auch eine Führungsdrahtaustrittstelle bzw. -öffnung vorgesehen ist, das proximale Schaftsegment mit einem distalen Schaftsegment verbunden ist, das insbesondere wiederum mit dem Ballon verbunden ist.

Weiterhin sind aus dem Stand der Technik dreigliedrige Schaftdesigns bekannt, bei denen das proximale Schaftsegment (z.B. Hypotube) zunächst in einen Stützschlauch und sodann über eine Verbindungsstelle, an der die Führungsdrahtaustrittsstelle bzw. der Sideport vorgesehen ist, in ein distales sowie koaxiales Schaftsegment übergeht.

So beschreibt z.B. die EP1084728A1 einen Katheter, bei dem eine vergleichsweise steife sowie abgeschrägte Hypotube über einen Übergangsbereich mit einer vergleichsweise weichen distalen Baugruppe verbunden ist.

Weiterhin beschreibt die US2010/0168669A1 einen Katheter, bei dem ein vergleichsweise starrer proximaler Schaftabschnitt mit einem distalen Schaftabschnitt über ein flexibles Übergangselement miteinander verbunden sind, wobei der distale Schaftabschnitt ein Führungsdrahtlumen aufweist. In US2010217234A1 und in EP1199082A1 werden Kathetersysteme mit Mandrel befestigt am Außenschaft beschrieben.

Hiervon ausgehend liegt der vorliegenden Erfindung die Aufgabe zugrunde, einen Katheter bereitzustellen, der eine verhältnismäßig einfach zu produzierende Führungsdrahtöffnung zum Herausführen eines Führungsdrahtes aus dem Katheter aufweist, so dass gleichzeitig eine geringe Knickempfindlichkeit im Bereich der Führungsdrahtöffnung gegeben ist.

Diese Aufgabe wird durch einen Katheter mit den Merkmalen des Anspruchs 1 gelöst. Vorteilhafte Ausgestaltungen der Erfindung sind in den entsprechenden Unteransprüchen angegeben und werden nachfolgend beschrieben.

Ein weiterer Aspekt der vorliegenden Erfindung betrifft ein Verfahren zur Herstellung eines erfindungsgemäßen Katheters mit den Merkmalen des Anspruchs 14.

Gemäß Anspruch 1 wird ein Katheter offenbart, aufweisend einen Außenschaft, der ein Lumen umgibt, wobei der Außenschaft einen proximalen Außenschaftabschnitt sowie einen damit verbundenen distalen Außenschaftabschnitt aufweist, wobei in einem vom distalen Außenschaftabschnitt umgebenen distalen Abschnitt des Lumens ein Innenschaft angeordnet ist, der ein Führungsdrahtlumen zur Aufnahme eines Führungsdrahts aufweist, und wobei der Innenschaft an einem proximalen Endabschnitt des Innenschaftes eine Öffnung aufweist, über die der Führungsdraht aus dem Außenschaft bzw. Katheter herausführbar ist.

Erfindungsgemäß ist vorgesehen, dass im Lumen des Außenschaftes ein metallisches Rohrelement angeordnet ist, über das der distale Abschnitt des Lumens mit einem vom proximalen Außenschaftabschnitt umgebenen proximalen Abschnitt des Lumens kommuniziert, wobei der proximale Endabschnitt des Innenschaftes mit dem Rohrelement in Eingriff steht, und wobei im Lumen ein separates, längs erstrecktes sowie metallisches Aussteifungselement angeordnet ist, das mit dem Rohrelement verbunden ist und zum abschnittsweisen Aussteifen des Außenschaftes vom Rohrelement in den distalen Abschnitt des Lumens sowie in den proximalen Abschnitt des Lumens absteht.

Gemäß einer Ausführungsform des erfindungsgemäßen Katheters ist vorgesehen, dass der proximale Außenschaftabschnitt mit dem distalen Außenschaftabschnitt über eine stoffschlüssige Verbindung, insbesondere eine Schweißverbindung, verbunden ist, wobei sich die Verbindung ein einer Umfangsrichtung des Rohrelements entlang einer Außenseite einer Wandung des Rohrelements erstreckt. Weiterhin sind die beiden Außenschaftabschnitte gemäß einer Ausführungsform auch mit der Außenseite der Wandung des Rohrelements verbunden, insbesondere über eine stoff-, form-, und/oder kraftschlüssige Verbindung.

Gemäß einer Ausführungsform des erfindungsgemäßen Katheters ist vorgesehen, dass sich der Innenschaft zumindest abschnittsweise koaxial im Lumen des distalen Außenschaftabschnitts erstreckt.

Bei dem Aussteifungselement handelt es sich gemäß einer Ausführungsform vorzugsweise um einen Draht aus einem Metall. Bei dem Metall kann es sich z.B. um Edelstahl wie AISI 304/ 302/ 316, oder Nickellegierungen wie Inconel oder Nimonic 90 handeln. Als weitere Materialien kommen Titan, Aluminium, Kupfer oder Legierungen daraus in Frage.

Insbesondere sind Kupferlegierungen wie CuBe₂ wegen ihrer Elastizität und Korrosionsunempfindlichkeit von Vorteil, da ein Aussteifungselement aus einem solchen Material weniger anfällig für Verbindungsdefekte beim anschließenden Krimpen eines Stents auf einen Ballon ist.

Weiterhin besteht das Rohrelement gemäß einer Ausführungsform ebenfalls aus einem Metall, insbesondere aus einem Edelstahl wie beispielsweise AISI 304/ 302/ 316.

Weiterhin ist gemäß einer Ausführungsform des erfindungsgemäßen Katheters vorgesehen, dass das Rohrelement einen proximalen Endabschnitt, einen distalen Endabschnitt sowie insbesondere eine in Umfangsrichtung des Rohrelements umlaufende Wandung aufweist, die ein Lumen des Rohrelements umgibt.

Weiterhin ist gemäß einer Ausführungsform des erfindungsgemäßen Katheters vorgesehen, dass das Rohrelement derart gekrümmt bzw. geformt ist, dass der distale Endabschnitt des Rohrelements versetzt zum proximalen Endabschnitt des Rohrelements verläuft. Vorzugsweise wird diese Krümmung durch ein Umformen des Rohrelements erzeugt.

Weiterhin ist erfindungsgemäß vorgesehen, dass das Aussteifungselement sich durch das Lumen des Rohrelements hindurch erstreckt und im Lumen des Rohrelements festgeklemmt ist, und zwar durch Umformen des Rohrelements, und, gemäß einer Ausführungsform, mit dem Rohrelement über eine Schweißverbindung verbunden ist.

Weiterhin ist erfindungsgemäß vorgesehen, dass die Öffnung des proximalen Endabschnitts des Innenschaftes an einer Außenseite des Außenschafts lateral zum Rohrelement angeordnet ist, wobei insbesondere die Öffnung an einer Stirnseite des proximalen Endabschnitts ausgebildet ist, d.h., an einem proximalen Ende des Innenschaftes. Eine solche Öffnung wird daher auch als Sideport bezeichnet.

Weiterhin ist gemäß einer Ausführungsform des erfindungsgemäßen Katheters vorgesehen, dass der distale Endabschnitt des Rohrelements an einer Außenseite der Wandung eine konkave Mulde aufweist, in der zumindest ein Teil des proximalen Endabschnitts des Innenschaftes eingreift bzw. angeordnet ist, wodurch der besagte Eingriff hergestellt wird.

Weiterhin ist gemäß einer Ausführungsform des erfindungsgemäßen Katheters vorgesehen, dass die Mulde durch Umformen der Wandung des Rohrelements in die Wandung eingebracht ist.

Weiterhin ist gemäß einer Ausführungsform des erfindungsgemäßen Katheters vorgesehen, dass das Aussteifungselement durch die Mulde im Lumen des Rohrelements eingeklemmt ist.

Weiterhin ist gemäß einer Ausführungsform des erfindungsgemäßen Katheters vorgesehen, dass der proximale Endabschnitt des Innenschaftes mit dem Rohrelement in Eingriff steht, indem der proximale Endabschnitt des Innenschaft in den distalen Endabschnitt des Rohrelements hereinsteht und sich im Lumen des Rohrelements durch den distalen Endabschnitt des Rohrelements hindurch erstreckt.

Weiterhin ist gemäß einer Ausführungsform des erfindungsgemäßen Katheters vorgesehen, dass die Wandung des Rohrelements in einem Verbindungsabschnitt bzw. in einem mittleren Abschnitt des Rohrelements, der den proximalen Endabschnitt des Rohrelements mit dem distalen Endabschnitt des Rohrelements verbindet, eine Durchgangsöffnung aufweist, durch die ein Teil des proximalen Endabschnitts des Innenschaftes aus dem Lumen des Rohrelements herausgeführt ist.

Weiterhin ist gemäß einer Ausführungsform des erfindungsgemäßen Katheters vorgesehen, dass sich die Durchgangsöffnung (bei linear angeordnetem Außenschaft) entlang einer normal zu einer Längsachse des Außenschaftes verlaufenden Richtung erstreckt, so dass sich der Innenschaft im Bereich der Durchgangsöffnung parallel zur Längsachse erstreckt und der Führungsdraht linear, d.h., ohne nennenswerte Krümmung, über die Öffnung des Innenschaftes aus dem Führungsdrahtlumen bzw. dem Außen- und Innenschaft herausführbar ist.

Weiterhin ist gemäß einer Ausführungsform des erfindungsgemäßen Katheters vorgesehen, dass das Aussteifungselement im proximalen Endabschnitt des Rohrelements im Lumen des Rohrelements festgeklemmt ist. Diesbezüglich kann vorgesehen sein, dass das Aussteifungselement insbesondere durch Umformen, insbesondere durch Abflachen oder Eindellen des proximalen Endabschnitts des Rohrelements im Lumen des Rohrelements festgeklemmt ist.

Weiterhin ist gemäß einer Ausführungsform des erfindungsgemäßen Katheters vorgesehen, dass der proximale Außenschaftabschnitt eine metallische Rohrstruktur (Hypotube) aufweist, die über ein flexibles Schlauchelement des proximalen Außenschaftabschnitts mit dem Rohrelement bzw. mit dem distalen Außenschaftabschnitt verbunden ist, wobei sich insbesondere das Aussteifungselement mit einem proximalen Endabschnitt (ausgehend vom Rohrelement) bis in einen Abschnitt des Lumens des Außenschaftes erstreckt, der von der metallischen Rohrstruktur umgeben ist.

Gemäß einer Ausführungsform der Erfindung ist weiterhin vorgesehen, dass das flexible Schlauchelement des proximalen Außenschaftabschnitts eine Wandung aufweist, die durch eines oder mehrere der folgenden Materialien gebildet ist oder eines oder mehrere der folgenden Materialien aufweist: ein Polymer, Polyamid, Polyurethan, Polyester wie Polyethylenterephthalat, Polyestercopolymere wie z.B. Hytrel (Markenname), Polyetherblockamide wie z.B. PEBAX (Markenname). Die zuvor genannten Materialien sind von daher bevorzugt, als dass mit diesen eine Schweiß- oder Schmelzverbindung zu einer breiten Materialpallette möglich ist.

Weiterhin ist gemäß einer Ausführungsform des erfindungsgemäßen Katheters vorgesehen, dass der distale Außenschaftabschnitt ein Schlauchelement aufweist, das mit dem Rohrelement bzw. mit dem proximalen Außenschaftabschnitt verbunden ist, wobei sich insbesondere das Aussteifungselement mit einem distalen Endabschnitt (ausgehend vom Rohrelement) bis in einen Abschnitt des Lumens des Außenschaftes erstreckt, der von dem Schlauchelement des distalen Außenschaftabschnitts umgeben ist.

Gemäß einer Ausführungsform der Erfindung ist weiterhin vorgesehen, dass das flexible Schlauchelement des distalen Außenschaftabschnitts eine Wandung aufweist, die durch eines oder mehrere der folgenden Materialien gebildet ist oder eines oder mehrere der folgenden Materialien aufweist: ein Polymer, Polyamid, Polyurethan, , Polyester wie Polyethylenterephthalat, Polyestercopolymere wie z.B. Hytrel (Markenname), Polyetherblockamide wie z.B. PEBAX (Markenname). Die zuvor genannten Materialien sind von daher bevorzugt, als dass mit diesen eine Schweiß- oder Schmelzverbindung zu einer breiten Materialpallette von Kunststoffen unterschiedlicher mechanischer Eigenschaften möglich ist.

Gemäß einer weiteren Ausführungsform der Erfindung ist vorgesehen, dass der distale Außenschaftabschnitt, insbesondere das Schlauchelement des distalen Außenschaftabschnitts an einem distalen Ende des distalen Außenschaftabschnitt mit einem enfaltbaren, insbesondere aufblasbaren, Ballon verbunden ist.

Ein weiterer Aspekt der vorliegenden Erfindung betrifft ein Verfahren zur Herstellung des Katheters (insbesondere Ballonkatheter).

Diesbezüglich wird ein Verfahren zur Herstellung eines Katheters offenbart (insbesondere eines erfindungsgemäßen Katheters), wobei ein proximaler Außenschaftabschnitt und ein distaler Außenschaftabschnitt des herzustellenden Katheters bzw. Außenschaftes bereitgestellt werden, wobei in einem vom distalen Schaftabschnitt umgebenen Abschnitt eines Lumens des herzustellenden Katheters ein Innenschaft angeordnet ist, der ein Führungsdrahtlumen zur Aufnahme eines Führungsdrahts aufweist, und wobei ein metallisches Rohrelement bereitgestellt wird, wobei ein längs erstrecktes sowie metallisches Aussteifungselement in einem Lumen des Rohrelements durch Umformen des Rohrelementes am Rohrelement festgelegt wird, wobei ein proximaler Endabschnitt des Innenschaftes mit dem Rohrelement in Eingriff gebracht wird, und wobei der proximale Außenschaftabschnitt und der distale Außenschaftabschnitt mit dem Rohrelement so verbunden werden, dass das Aussteifungselement vom Rohrelement in einen vom proximalen Außenschaftabschnitt umgebenen proximalen Abschnitt des Lumens des Außenschaftes und in den distalen Abschnitt des Lumens des Außenschaftes geführt ist.

Das besagte Umformen erfolgt gemäß einer Ausführungsform des erfindungsgemäßen Verfahrens mittels einer Matrize und mittels eines Stempels.

Gemäß einer Ausführungsform des erfindungsgemäßen Verfahrens ist vorgesehen, dass das Rohrelement vor dem Verbinden mit dem distalen und dem proximalen Außenschaftabschnitt derart gekrümmt wird, vorzugsweise durch ein Umformen des Rohrelements, vorzugsweise mittels der Matrize und mittels des Stempels, dass der distale Endabschnitt des Rohrelements versetzt zu dem proximalen Endabschnitt des Rohrelements verläuft.

Weiterhin ist gemäß einer Ausführungsform des erfindungsgemäßen Verfahrens vorgesehen, dass eine Öffnung des proximalen Endabschnitts des Innenschaftes, über die ein Führungsdraht aus dem Führungsdrahtlumen bzw. dem Katheter herausführbar ist, lateral zum Rohrelement angeordnet wird, wobei insbesondere die Öffnung an einer Stirnseite des proximalen Endabschnitts ausgebildet ist, d.h., an einem proximalen Ende des Innenschaftes (siehe auch oben).

Weiterhin ist gemäß einer Ausführungsform des erfindungsgemäßen Verfahrens vorgesehen, dass ein distaler Endabschnitt des Rohrelements so umgeformt wird, vorzugsweise mittels der besagten Matrize und mittels des besagten Stempels, dass der distale Endabschnitt des Rohrelements an einer Außenseite einer Wandung des Rohrelements eine konkave Mulde erhält, wobei insbesondere der besagte Eingriff zwischen Innenschaft und Rohrelement hergestellt wird, indem zumindest ein Teil des proximalen Endabschnitts des Innenschaftes in der Mulde angeordnet wird.

Weiterhin ist gemäß einer Ausführungsform des erfindungsgemäßen Verfahrens vorgesehen, dass das Aussteifungselement durch die Mulde im Lumen des Rohrelements eingeklemmt wird (siehe auch oben).

Weiterhin ist gemäß einer alternativen Ausführungsform des erfindungsgemäßen Verfahrens vorgesehen, dass der proximale Endabschnitt des Innenschaftes mit dem Rohrelement in Eingriff gebracht wird, indem der proximale Endabschnitt des Innenschaft in den distalen Endabschnitt des Rohrelements eingeführt wird, so dass er sich im Lumen des Rohrelements durch den distalen Endabschnitt des Rohrelements hindurch erstreckt.

Weiterhin ist diesbezüglich gemäß einer Ausführungsform des erfindungsgemäßen Verfahrens vorgesehen, dass in der Wandung des Rohrelements in einem Verbindungsabschnitt bzw. in einem mittleren Abschnitt des Rohrelements, der einen proximalen Endabschnitt des Rohrelements mit dem distalen Endabschnitt des Rohrelements verbindet, eine Durchgangsöffnung ausgebildet wird, durch die ein Teil des proximale Endabschnitts des Innenschaftes aus dem Lumen des Rohrelements herausgeführt wird.

Weiterhin ist gemäß einer Ausführungsform des erfindungsgemäßen Verfahrens vorgesehen, dass das Aussteifungselement im proximalen Endabschnitt des Rohrelements im Lumen des Rohrelements festgeklemmt wird, insbesondere durch Umformen, insbesondere durch Abflachen oder Eindellen des proximalen Endabschnitts des Rohrelements.

Weitere Merkmale und Ausführungsformen der vorliegenden Erfindung sollen nachfolgend im Zusammenhang mit den Figuren beschrieben werden. Es zeigen:
- Fig. 1: eine schematische Schnittdarstellung einer Ausführungsform eines erfindungsgemäßen Katheters, hier in Form eines Ballonkatheters;
- Fig. 2: eine schematische Schnittdarstellung einer Ausführungsform eines Rohrelements eines erfindungsgemäßen Katheters;
- Fig. 3: eine schematische Schnittdarstellung einer weiteren Ausführungsform eines Rohrelements eines erfindungsgemäßen Katheters; und
- Fig. 4: eine perspektivische Ansicht einer Matrize und eines Stempels zum Umformen des Rohrelements bei der Herstellung eines erfindungsgemäßen Katheters, ohne Rohrelement (A) sowie mit Rohrelement (B).

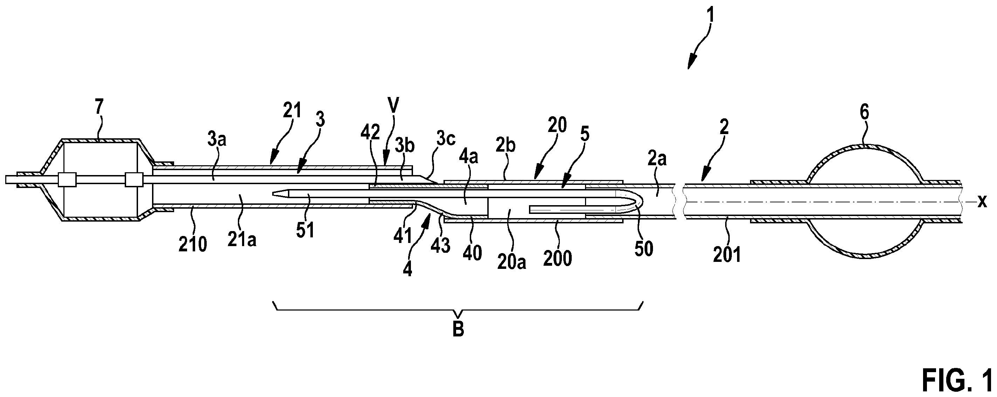

Fig. 1 zeigt eine schematische Schnittansicht eines erfindungsgemäßen Katheters 1, mit einem längs erstreckten Außenschaft 2, wobei der Außenschaft 2 ein Lumen 2a des Katheters 1 umgibt und ferner einen proximalen Außenschaftabschnitt 20 sowie einen mit dem proximalen Außenschaftabschnitt 20 verbundenen distalen Außenschaftabschnitt 21 aufweist. Die beiden Außenschaftabschnitte 20, 21 umgeben jeweils einen Abschnitt 20a bzw. 21a des Lumens 2a, wobei die beiden Abschnitte 20a, 21a des Lumens 2a miteinander kommunizieren, d.h., mit einander strömungsverbunden sind. Weiterhin ist im distalen Abschnitt 21a des Lumens 2a ein Innenschaft 3 angeordnet, der ein Führungsdrahtlumen 3a zur Aufnahme eines Führungsdrahts 30 aufweist, wobei der Innenschaft 3 an einem proximalen Endabschnitt 3b des Innenschaftes 3 eine Öffnung 3c aufweist, über die der Führungsdraht 30 aus dem Außenschaft 2 herausführbar ist. Der Innenschaft 3 erstreckt sich gemäß einer Ausführungsform zumindest abschnittsweise koaxial im distalen Abschnitt 21a des Lumen 2a.

Der Katheter 1 ist vorliegend als Ballonkatheter ausgestaltet und weist entsprechend einen mit dem distalen Außenschaftabschnitt 21 verbundenen Ballon 7 auf, der z.B. mittels Druckluft aufblasbar ist, die über die Abschnitte 20a bzw. 21a des Lumens 2a in den Ballon 7 einleitbar ist. Der Innenschaft 3 erstreckt sich durch den Ballon 7 hindurch, so dass der Ballon 7 in einem Gefäß eines Patienten mittels des Führungsdrahts 30 zu einem Einsatzort führbar ist.

Erfindungsgemäß weist der Katheter 1 ein metallisches Rohrelement 4 auf, das im Lumen 2a des Außenschaftes 2 angeordnet ist, so dass der distale Abschnitt 21a und der proximale Abschnitt 20a des Lumens 2a über ein Lumen 4a des Rohrelements 4 kommunizieren, das von einer Wandung 43 des Rohrelements 4 umgeben ist. D.h., zum Aufblasen des Ballons 7 kann die Druckluft oder ein anderes geeignetes Strömungsmittel in den proximalen Abschnitt 20a des Lumens 2a eingeleitet werden, strömt durch das Lumen 4a des Rohrelements 4 hindurch und gelangt über den sich daran anschließenden Abschnitt 21a des Lumens 2a in den Ballon 7.

Der distale und der proximale Außenschaftabschnitt 21, 20 sind weiterhin im Bereich einer Außenseite 43a des Rohrelements 4 miteinander verbunden, insbesondere stoffschlüssig, z.B. indem die beiden Abschnitte 20, 21 miteinander verschweißt sind. Weiterhin sind die beiden Abschnitte 20, 21 des Außenschaftes 2 stoffschlüssig und/oder formschlüssig und/oder kraftschlüssig mit der Außenseite 43a der Wandung 43 des Rohrelements 4 verbunden.

Der Katheter 1 weist weiterhin zum abschnittsweisen Aussteifen des Außenschaftes 2 im Bereich der lateralen Öffnung 3c des Innenschaftes 3 (in der Figur 1 als Übergangsbereich B gekennzeichnet) ein Aussteifungselement 5 auf, insbesondere in Form eines separaten Metalldrahts, der an dem Rohrelement 4 festgelegt ist, insbesondere durch Festklemmen des Aussteifungselementes 5 im Rohrelement 4 und/oder mittels einer stoffschlüssigen Verbindung (z.B. Schweißverbindung). Das Aussteifungselement 5 ist insbesondere solchermaßen im Lumen 4a des Rohrelements 4 angeordnet, dass es zum Aussteifen des Übergangsbereichs B in den distalen Abschnitt 21a des Lumens 2a sowie in den proximalen Abschnitt 20a des Lumens 2a absteht.

Die Öffnung 3c bildet vorzugsweise einen sogenannten Sideport und ist entsprechend lateral am Katheter 1 angeordnet, d.h., an einer Außenseite 2b des Außenschafts 2. Hierbei ist die Öffnung 3c vorzugsweise an einem Übergang zwischen den beiden Außenschaftabschnitten 20, 21 vorgesehen, der an der Außenseite 43a des Rohrelements 4 anliegt.

Der proximale Endabschnitt 3b des Innenschaftes 3 wird vorzugsweise beim Verbinden der beiden Außenschaftabschnitte 20, 21 abdichtend im Außenschaft 2 eingeschlossen.

Wie in der Figur 1 angedeutet, weist das Rohrelement 4 einen proximalen Endabschnitt 40 und einen distalen Endabschnitt 42 auf, die insbesondere über einen Verbindungsabschnitt 41 miteinander verbunden sind. Dabei kann das Rohrelement 4, insbesondere der Verbindungs- bzw. mittlere Abschnitt 41, derart gekrümmt sein, dass der distale Endabschnitt 42 des Rohrelements 4 - bezüglich der Längsachse x des Außenschafts 2 - versetzt zum proximalen Endabschnitt 40 des Rohrelements 4 verläuft.

Weiterhin weist der proximale Außenschaftabschnitt 20 vorzugsweise eine metallische Rohrstruktur 201 auf, die auch als Hypotube bezeichnet wird und die über ein flexibles Schlauchelement 200 des proximalen Außenschaftabschnitts 20 mit dem Rohrelement 4 bzw. einem flexiblen Schlauchelement 210 des distalen Außenschaftabschnitt 42 verbunden ist (über die oben beschriebene z.B. stoffschlüssige Verbindung). Die metallische Rohrstruktur 201 ist weiterhin insbesondere an einem Griff 6 zum Handhaben des Katheters 1 festgelegt. Hinsichtlich der Rohrstruktur 201 ist weiterhin vorgesehen, dass sich das Aussteifungselement 5 mit einem proximalen Endabschnitt 50 bis in einen Abschnitt des Lumens 2a erstreckt, der von der metallischen Rohrstruktur 201 umgeben ist.

Weiterhin ist ebenfalls bevorzugt vorgesehen, dass sich das Aussteifungselement 5 mit einem distalen Endabschnitt 51 bis in einen Abschnitt des Lumens 2a erstreckt, der von dem Schlauchelement 210 des distalen Außenschaftabschnitts 21 umgeben ist.

Hierdurch wird im Bereich der lateralen Öffnung 3c eine hinreichende Aussteifung des Außenschaftes 2 erzeugt.

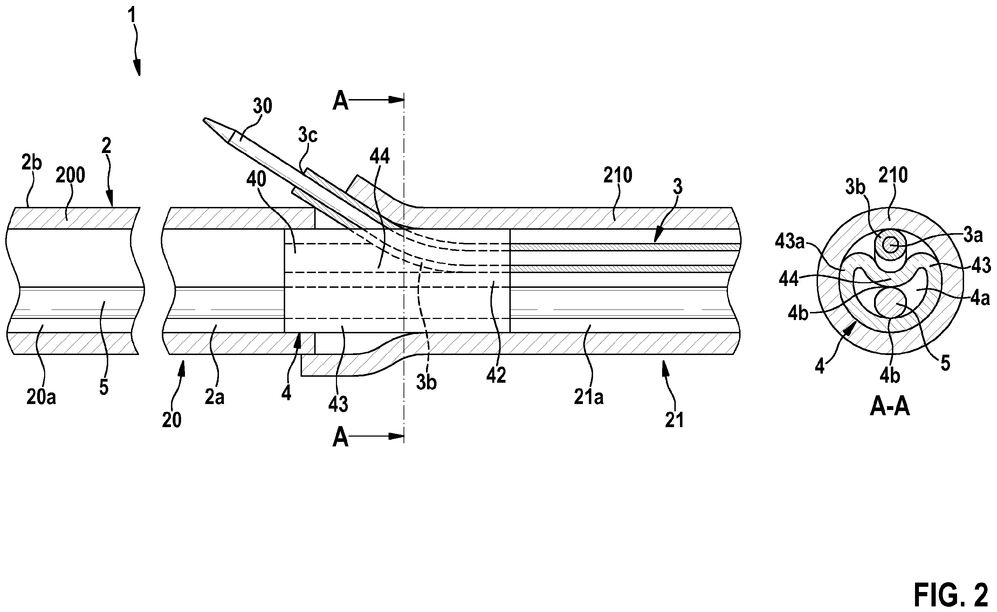

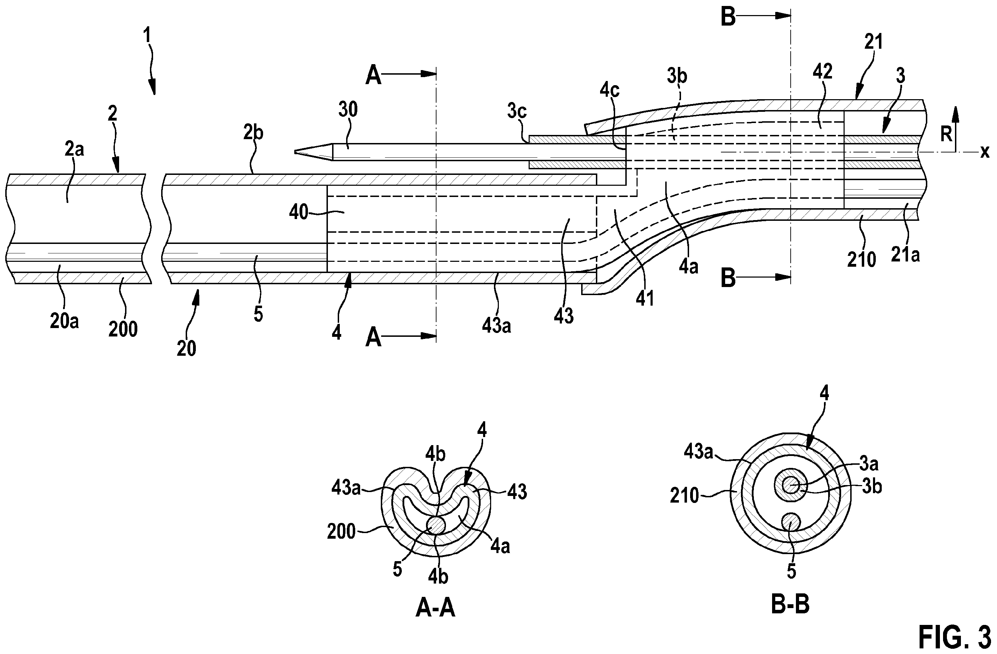

Die Figuren 2 und 3 zeigen im Detail zwei unterschiedliche Ausführungsformen hinsichtlich der näheren Ausgestaltung des Rohrelements 4.

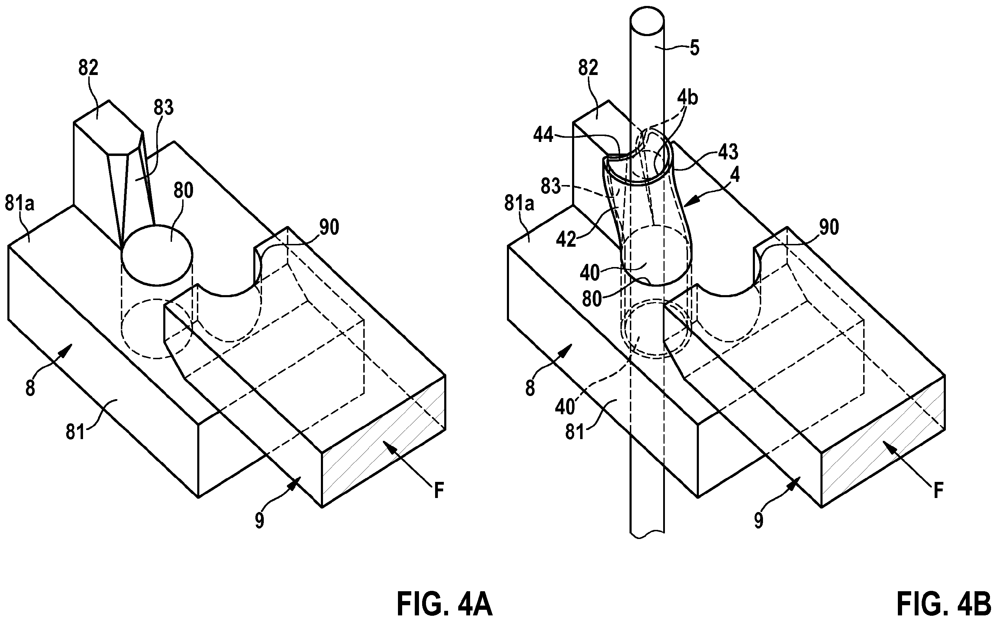

Nach der in der Figur 2 darstellten Variante ist vorgesehen, dass der distale Endabschnitt 42 des Rohrelements 4 an einer Außenseite 43a der Wandung 43 eine konkave Mulde 44 aufweist, in der zumindest ein Teil des proximalen Endabschnitts 3b des Innenschaftes 3 eingreift bzw. angeordnet ist. Die Mulde 44 ist vorzugsweise durch Umformen der Wandung 43 des Rohrelements 4 in die Wandung 43 eingebracht. Das Umformen kann z.B. mit Hilfe des in der Figur 4 gezeigten Werkzeugs erfolgen, dass eine Matritze 8 sowie einen Stempel 9 zum Umformen des Rohrelements 4 aufweist.

Durch das Umformen wird auch das Aussteifungselement 5 durch die Mulde 44 im distalen Endabschnitt 42 im Lumen 4a des Rohrelements 4 eingeklemmt, indem durch die Ausformung der Mulde 44 zwei einander gegenüberliegende Bereiche 4b der Innenseite des Rohrelements 4 gegen das Aussteifungselement 5 drücken. Figur 1 zeigt den Ort dieser Verbindung entlang der Längsachse x als Verbindungsstelle V.

Weiterhin ist bevorzugt vorgesehen, dass der proximale und der distale Außenschaftabschnitt 20, 21 in der gezeigten Querschnittsebene A der Fig. 2 überlappt werden und miteinander verschweißt werden, wobei der proximale Endabschnitt 3b des Innenschaftes 3 abdichtend zwischen den beiden Abschnitten 20, 21 des Außenschaftes 2 eingeschlossen wird.

Gemäß Figur 2 ist das Rohrelement 4, abgesehen von der Mulde 44, im Wesentlichen zylindrisch ausgebildet, kann jedoch auch die in der Figur 1 gezeigten Krümmung (siehe oben) aufweisen.

Fig. 3 zeigt eine alternative Ausgestaltung des Rohrelementes 4. Hier ist insbesondere der Verbindungsabschnitt 41 des Rohrelements 4 solchermaßen umgeformt bzw. gekrümmt, dass der proximale Endabschnitt 40 und der distale Endabschnitt 42 versetzt zueinander verlaufen. Weiterhin ist in dem Verbindungsabschnitt 41 eine Durchgangsöffnung 4c in der Wandung 43 ausgebildet, die sich entlang einer senkrecht zur Längsachse x orientierten Richtung R erstreckt. Das schließt auch die Möglichkeit ein, dass die Öffnungsebene der Durchgangsöffnung 4c geneigt zur Längsachse x verläuft.

Gemäß Fig. 3 ist vorgesehen, dass der proximale Endabschnitt 3b des Innenschaftes 3 mit dem Rohrelement 4 in Eingriff steht, indem der proximale Endabschnitt 3b des Innenschaftes 3 in den distalen Endabschnitt 42 des Rohrelements 4 hereinsteht und sich im Lumen 4a des Rohrelements 4 durch den distalen Endabschnitt 42 des Rohrelements 4 hindurch erstreckt, und teilweise durch die Durchgangsöffnung 4c aus dem Rohrelement 4 sowie insbesondere dem Außenschaft 2 herausgeführt ist.

Aufgrund der Anordnung der Durchgangsöffnung 4c bzw. der Krümmung des Rohrelements 4 ist es möglich, dass sich der Innenschaft 3 im Bereich der Durchgangsöffnung 4c parallel zur Längsachse x des Außenschafts 2 erstrecken kann und der Führungsdraht 30 entsprechend linear, d.h., ohne nennenswerte Krümmung, über die Öffnung 3c des Innenschaftes 3 aus dem Führungsdrahtlumen 3a bzw. dem Außen- und Innenschaft 2, 3 herausführbar ist.

Im Unterschied zu der in der Figur 2 gezeigten Variante ist vorgesehen, dass das Aussteifungselement 5 im proximalen Endabschnitt 40 des Rohrelements 4 im Lumen 4a des Rohrelements 4 festgeklemmt ist. Figur 2 zeigt diesbezüglich einen Schnitt entlang der Ebene A. Danach wird der proximale Endabschnitt 40 des Rohrelements 4 zum Festklemmen des Rohrelements 4 umgeformt, z.B. durch Eindellen oder Abflachen der Wandung 43, so dass wiederum zwei einander gegenüberliegende Bereiche 4b der Innenseite der Wandung 43 des Rohrelements gegen das Aussteifungselement 5 drücken.

Das Aussteifungselement 5 kann in allen Ausführungsformen alternativ oder zusätzlich auch über eine Schweißverbindung mit dem Rohrelement 4 verbunden sein.

Die im Zusammenhang mit den Figuren 1 und 2 beschriebene Mulde 44 kann insbesondere mittels des in der Figur 4 gezeigten Werkzeugs geformt werden. Das Werkzeug weist eine Matritze 8 auf sowie einen zugehörigen Stempel 9 auf.

Gemäß Figur 4(A) weist die Matritze 8 eine zylindrische Durchgangsöffnung 80 auf, die in einer Basis 81 der Matritze 8 ausgebildet ist, wobei von einem Rand der Öffnung 80 ein Vorsprung 82 von der Basis 81 absteht. Der Vorsprung weist dabei eine Außenseite 83 auf, die eine positive Form für die zu formende Mulde 44 bildet. Zur Ausbildung der Mulde 44 sowie insbesondere zur Ausbildung einer Krümmung des Verbindungsabschnitts 41 des Rohrelements 4, wird gemäß Figur 4(B) ein zylindrischer Rohling 4 in die Durchgangsöffnung 80 eingeführt, so dass der spätere proximale Endabschnitt des Rohrelements in der Durchgangsöffnung 80 angeordnet ist. Weiterhin wird das Aussteifungselement 5 in das Lumen 4a des Rohlings 4 bzw. Rohrelements 4 eingelegt. Sodann wird der Stempel 9 entlang einer Oberfläche 81a der Basis 81 in Richtung auf den Vorsprung 82 verfahren, wobei eine Stirnseite 90 des Stempels 9 in Eingriff mit dem Rohling 4 gelangt und diesen mit einer definierten Kraft F gegen die Außenseite 83 des Vorsprung 82 drückt. Hierbei legt sich die Wandung 43 des Rohlings 4 im Bereich des distalen Endabschnitts 4 des Rohlings 4 um die Außenseite 83 bzw. den Vorsprung 82 unter Ausbildung der Mulde 44 sowie insbesondere der besagten Krümmung des Rohrelements 4 herum. Gleichzeitig wird der aus der Durchgangsöffnung 80 herausstehende distale Endabschnitt 42 des Rohrelements 4 aufgrund der Ausformung der Mulde 44 im Lumen 4a des Rohrelements 4 eingeklemmt (siehe auch oben).

## Patentansprüche

1. Katheter (1), aufweisend einen Außenschaft (2), der ein Lumen (2a) umgibt, wobei der Außenschaft (2) einen proximalen Außenschaftabschnitt (20) sowie einen damit verbundenen distalen Außenschaftabschnitt (21) aufweist, wobei in einem vom distalen Außenschaftabschnitt (21) umgebenen distalen Abschnitt (20a) des Lumens (2a) ein Innenschaft (3) angeordnet ist, der ein Führungsdrahtlumen (3a) zur Aufnahme eines Führungsdrahts (30) aufweist, und wobei der Innenschaft (3) an einem proximalen Endabschnitt (3b) des Innenschaftes (3) eine Öffnung (3c) aufweist, über die der Führungsdraht (30) aus dem Außenschaft (2) herausführbar ist, wobei im Lumen (2a) des Außenschaftes (2) ein metallisches Rohrelement (4) angeordnet ist, über das der distale Abschnitt (21a) des Lumens (2a) mit einem vom proximalen Außenschaftabschnitt (20) umgebenen proximalen Abschnitt (20a) des Lumens (2a) kommuniziert, wobei der proximale Endabschnitt (3b) des Innenschaftes (3) mit dem Rohrelement (4) in Eingriff steht, und wobei im Lumen (2a) ein separates, längs erstrecktes sowie metallisches Aussteifungselement (5) angeordnet ist, das mit dem Rohrelement (4) verbunden ist und zum abschnittsweisen Aussteifen des Außenschaftes (2) vom Rohrelement (4) in den distalen Abschnitt (21a) des Lumens (2a) sowie in den proximalen Abschnitt (20a) des Lumens (2a) absteht,
**dadurch gekennzeichnet, dass**
das Aussteifungselement (5) sich durch das Lumen (4a) des Rohrelements (4) hindurch erstreckt und im Lumen (4a) des Rohrelements (4) festgeklemmt ist und dass die Öffnung (3c) des proximalen Endabschnitts (3b) des Innenschaftes (3) an einer Außenseite (2b) des Außenschafts (2) lateral zum Rohrelement (4) angeordnet ist.

2. Katheter nach Anspruch 1, **dadurch gekennzeichnet, dass** das Rohrelement (4) einen proximalen Endabschnitt (40), einen distalen Endabschnitt (42) sowie eine Wandung (43) aufweist, die ein Lumen (4a) des Rohrelements (4) umgibt.

3. Katheter nach Anspruch 2, **dadurch gekennzeichnet, dass** das Rohrelement (4) derart gekrümmt ist, dass der distale Endabschnitt (42) des Rohrelements (4) versetzt zum proximalen Endabschnitt (40) des Rohrelements (4) verläuft.

4. Katheter nach Anspruch 3, **dadurch gekennzeichnet, dass** das Aussteifungselement (5) sich durch das Lumen (4a) des Rohrelements (4) hindurch erstreckt und mit dem Rohrelement (4) über eine Schweißverbindung verbunden ist.

5. Katheter nach einem der Ansprüche 2 bis 4, **dadurch gekennzeichnet, dass** der distale Endabschnitt (42) des Rohrelements (4) an einer Außenseite (43a) der Wandung (43) eine konkave Mulde (44) aufweist, in der zumindest ein Teil des proximalen Endabschnitts (3b) des Innenschaftes (3) eingreift.

6. Katheter nach Anspruch 5, **dadurch gekennzeichnet, dass** die Mulde (44) durch Umformen der Wandung (43) des Rohrelements (4) in die Wandung (43) eingebracht ist.

7. Katheter nach einem der Ansprüche 1, 4, 5 oder 6, **dadurch gekennzeichnet, dass** das Aussteifungselement (5) durch die Mulde (44) im Lumen (4a) des Rohrelements (4) eingeklemmt ist.

8. Katheter nach einem der Ansprüche 2 bis 4, **dadurch gekennzeichnet, dass** der proximale Endabschnitt (3b) des Innenschaftes (3) mit dem Rohrelement (4) in Eingriff steht, indem der proximale Endabschnitt (3b) des Innenschafts (3) in den distalen Endabschnitt (42) des Rohrelements (4) hereinsteht und sich im Lumen (4a) des Rohrelements (4) durch den distalen Endabschnitt (42) des Rohrelements (4) hindurch erstreckt.

9. Katheter nach Anspruch einem der Ansprüche 2 bis 4 oder nach Anspruch 8, **dadurch gekennzeichnet, dass** die Wandung (43) des Rohrelements (4) in einem Verbindungsabschnitt (41) des Rohrelements (4), der den proximalen Endabschnitt (40) des Rohrelements (4) mit dem distalen Endabschnitt (42) des Rohrelements (4) verbindet, eine Durchgangsöffnung (4c) aufweist, durch die ein Teil des proximalen Endabschnitts (3b) des Innenschaftes (3) aus dem Lumen (4a) des Rohrelements (4) herausgeführt ist.

10. Katheter nach Anspruch 9, **dadurch gekennzeichnet, dass** sich die Durchgangsöffnung (4c) entlang einer normal zu einer Längsachse (x) des Außenschaftes (2) verlaufenden Richtung (R) erstreckt.

11. Katheter nach einem der Ansprüche 2 bis 4 oder nach einem der Ansprüche 8 bis 10, **dadurch gekennzeichnet, dass** das Aussteifungselement (5) im proximalen Endabschnitt (40) des Rohrelements (4) im Lumen (4a) des Rohrelements (4) festgeklemmt ist.

12. Katheter nach einem der vorhergehenden Ansprüche, **dadurch gekennzeichnet, dass** der proximale Außenschaftabschnitt (20) eine metallische Rohrstruktur (201) aufweist, die über ein flexibles Schlauchelement (200) des proximalen Außenschaftabschnitts (20) mit dem Rohrelement (4) und/oder mit einem flexiblen Schlauchelement (210) des distalen Außenschaftabschnitts (21) verbunden ist, wobei sich insbesondere das Aussteifungselement (5) mit einem proximalen Endabschnitt (50) bis in einen Abschnitt des Lumens (2a) erstreckt, der von der metallischen Rohrstruktur (201) umgeben ist.

13. Katheter nach einem der vorhergehenden Ansprüche, **dadurch gekennzeichnet, dass** der distale Außenschaftabschnitt (21) ein Schlauchelement (210) aufweist, das mit dem Rohrelement (4) und/oder mit einem flexiblen Schlauchelement (200) des proximalen Außenschaftabschnitts (20) verbunden ist, wobei sich insbesondere das Aussteifungselement (5) mit einem distalen Endabschnitt (51) bis in einen Abschnitt des Lumens (2a) erstreckt, der von dem Schlauchelement (210) des distalen Außenschaftabschnitts (21) umgeben ist.

14. Verfahren zur Herstellung eines Katheters (1), insbesondere nach einem der vorhergehenden Ansprüche, wobei ein proximaler Außenschaftabschnitt (20) und ein distaler Außenschaftabschnitt (21) des herzustellenden Katheters (1) bereitgestellt werden, wobei in einem vom distalen Schaftabschnitt (21) umgebenen distalen Abschnitt (21a) eines Lumens (2a) des herzustellenden Katheters (1) ein Innenschaft (3) angeordnet ist, der ein Führungsdrahtlumen (3a) zur Aufnahme eines Führungsdrahts (30) aufweist, und wobei ein metallisches Rohrelement (4) bereitgestellt wird, wobei ein längs erstrecktes sowie metallisches Aussteifungselement (5) in einem Lumen (4a) des Rohrelements (4) durch Umformen des Rohrelementes (4) festgelegt wird, wobei ein proximaler Endabschnitt (3b) des Innenschaftes (3) mit dem Rohrelement (4) in Eingriff gebracht wird, und wobei der proximale Außenschaftabschnitt (20) und der distale Außenschaftabschnitt (21) mit dem Rohrelement (4) so verbunden werden, dass das Aussteifungselement (5) vom Rohrelement (4) in einen vom proximalen Außenschaftabschnitt (20) umgebenen proximalen Abschnitt (20a) des Lumens (2a) und in den distalen Abschnitt (21a) des Lumens (2a) geführt ist.

## Claims

1. Catheter (1), comprising an outer shaft (2) surrounding a lumen (2a), wherein the outer shaft (2) has a proximal outer shaft portion (20) and a distal outer shaft portion (21) connected thereto, wherein an inner shaft (3) is arranged in a distal portion (20a) of the lumen (2a) surrounded by the distal outer shaft portion (21), the inner shaft (3) having a guide wire lumen (3a) for receiving a guide wire (30), and wherein the inner shaft (3) at a proximal end portion (3b) of the inner shaft (3) has an opening (3c) via which the guide wire (30) can be guided out from the outer shaft (2), wherein a metallic tube (4) is arranged in the lumen (2a) of the outer shaft (2), via which the distal portion (21a) of the lumen (2a) communicates with a proximal portion (20a) of the lumen (2a) surrounded by the proximal outer shaft portion (20), wherein the proximal end portion (3b) of the inner shaft (3) is engaged with the tube (4), and wherein a separate, elongate and metallic stiffening element (5) is arranged in the lumen (2a), which stiffening element is connected to the tube (4) and protrudes from the tube (4) into the distal portion (21a) of the lumen (2a) and into the proximal portion (20a) of the lumen (2a) for sectionwise stiffening the outer shaft (2),
**characterized in that**
the stiffening element (5) extends through the lumen (4a) of the tube (4) and is securely clamped in the lumen (4a) of the tube (4), and that the opening (3c) of the proximal end portion (3b) of the inner shaft (3) is arranged on an outer side (2b) of the outer shaft (2) laterally of the tube (4).

2. Catheter according to claim 1, **characterized in that** the tube (4) has a proximal end portion (40), a distal end portion (42) and a wall (43) surrounding a lumen (4a) of the tube (4).

3. Catheter according to claim 2, **characterized in that** the tube (4) is curved such that the distal end portion (42) of the tube (4) extends offset relative to the proximal end portion (40) of the tube (4).

4. Catheter according to claim 3, **characterized in that** the stiffening element (5) extends through the lumen (4a) of the tube (4) and is connected to the tube (4) via a welded connection.

5. Catheter according to any one of claims 2 to 4, **characterized in that** the distal end portion (42) of the tube (4) has, on an outer side (43a) of the wall (43), a concave hollow (44) in which at least part of the proximal end portion (3b) of the inner shaft (3) engages.

6. Catheter according to claim 5, **characterized in that** the hollow (44) is formed in the wall (43) by reshaping the wall (43) of the tube (4).

7. Catheter according to any one of claims 1, 4, 5 or 6, **characterized in that** the stiffening element (5) is clamped in the lumen (4a) of the tube (4) by the hollow (44).

8. Catheter according to any one of claims 2 to 4, **characterized in that** the proximal end portion (3b) of the inner shaft (3) is engaged with the tube (4) such that the proximal end portion (3b) of the inner shaft (3) protrudes into the distal end portion (42) of the tube (4) and extends in the lumen (4a) of the tube (4) through the distal end portion (42) of the tube (4).

9. Catheter according to any one of claims 2 to 4 or according to claim 8, **characterized in that** the wall (43) of the tube (4), in a connection portion (41) of the tube (4) connecting the proximal end portion (40) of the tube (4) to the distal end portion (42) of the tube (4), has a through-opening (4c) through which part of the proximal end portion (3b) of the inner shaft (3) is guided out from the lumen (4a) of the tube (4).

10. Catheter according to claim 9, **characterized in that** the through-opening (4c) extends along a direction (R) running perpendicular to a longitudinal axis (x) of the outer shaft (2).

11. Catheter according to any one of claims 2 to 4 or according to any one of claims 8 to 10, **characterized in that** the stiffening element (5) is securely clamped in the lumen (4a) of the tube (4) in the proximal end portion (40) of the tube (4).

12. Catheter according to any one of the preceding claims, **characterized in that** the proximal outer shaft portion (20) has a metallic tube structure (201), which is connected via a flexible hose element (200) of the proximal outer shaft portion (20) to the tube (4) and/or to a flexible hose element (210) of the distal outer shaft portion (21), wherein in particular the stiffening element (5) extends with a proximal end portion (50) into a portion of the lumen (2a) surrounded by the metallic tube structure (201).

13. Catheter according to any one of the preceding claims, **characterized in that** the distal outer shaft portion (21) has a hose element (210) which is connected to the tube (4) and/or to a flexible hose element (200) of the proximal outer shaft portion (20), wherein in particular the stiffening element (5) extends with a distal end portion (51) into a portion of the lumen (2a) surrounded by the hose element (210) of the distal outer shaft portion (21).

14. Method for producing a catheter (1), in particular according to any one of the preceding claims, wherein a proximal outer shaft portion (20) and a distal outer shaft portion (21) of the catheter (1) to be produced are provided, wherein an inner shaft (3) is arranged in a distal portion (21a) of a lumen (2a) of the catheter (1) to be produced, which distal portion is surrounded by the distal shaft portion (21), the inner shaft (3) having a guide wire lumen (3a) for receiving a guide wire (30), and wherein a metallic tube (4) is provided, wherein an elongate and metallic stiffening element (5) is secured in a lumen (4a) of the tube (4) by reshaping the tube (4), wherein a proximal end portion (3b) of the inner shaft (3) is brought into engagement with the tube (4), and wherein the proximal outer shaft portion (20) and the distal outer shaft portion (21) are connected to the tube (4) such that the stiffening element (5) is guided from the tube (4) into a proximal portion (20a) of the lumen (2a) surrounded by the proximal outer shaft portion (20) and into the distal portion (21a) of the lumen (2a).

## Revendications

1. Cathéter (1), présentant une tige extérieure (2) qui entoure une lumière (2a), dans lequel la tige extérieure (2) présente une section proximale de tige extérieure (20) ainsi qu'une section distale de tige extérieure (21) reliée à celle-ci, dans lequel une tige intérieure (3) présentant une lumière pour fil de guidage (3a) permettant d'accueillir un fil de guidage (30) est agencée dans une section distale (20a) de la lumière (2a) entourée par la section distale de tige extérieure (21), et dans lequel la tige intérieure (3) présente au niveau d'une section d'extrémité proximale (3b) de la tige intérieure (3) un orifice (3c) par l'intermédiaire duquel le fil de guidage (30) peut être guidé hors de la tige extérieure (2), dans lequel un élément tubulaire métallique (4), par l'intermédiaire duquel la section distale (21a) de la lumière (2a) communique avec une section proximale (20a) de la lumière (2a) entourée par la section proximale de tige extérieure (20), est agencé dans la lumière (2a) de la tige extérieure (2), dans lequel la section d'extrémité proximale (3b) de la tige intérieure (3) est en prise avec l'élément tubulaire (4), et dans lequel un élément de raidissement (5) séparé, allongé longitudinalement et métallique, est agencé dans la lumière (2a), ledit élément de raidissement étant relié à l'élément tubulaire (4) et faisant saillie de l'élément tubulaire (4) jusque dans la section distale (21a) de la lumière (2a) et dans la section proximale (20a) de la lumière (2a) afin de raidir par sections la tige extérieure (2),
**caractérisé en ce que**
l'élément de raidissement (5) s'étend à travers la lumière (4a) de l'élément tubulaire (4) et est calé dans la lumière (4a) de l'élément tubulaire (4), et **en ce que** l'orifice (3c) de la section d'extrémité proximale (3b) de la tige intérieure (3) est agencé latéralement par rapport à l'élément tubulaire (4) au niveau d'un côté extérieur (2b) de la tige extérieure (2).

2. Cathéter selon la revendication 1, **caractérisé en ce que** l'élément tubulaire (4) présente une section d'extrémité proximale (40), une section d'extrémité distale (42) et une paroi (43) qui entoure une lumière (4a) de l'élément tubulaire (4).

3. Cathéter selon la revendication 2, **caractérisé en ce que** l'élément tubulaire (4) est incurvé de façon telle que la section d'extrémité distale (42) de l'élément tubulaire (4) est décalée par rapport à la section d'extrémité proximale (40) de l'élément tubulaire (4).

4. Cathéter selon la revendication 3, **caractérisé en ce que** l'élément de raidissement (5) s'étend à travers la lumière (4a) de l'élément tubulaire (4) et est relié à l'élément tubulaire (4) par une liaison soudée.

5. Cathéter selon l'une des revendications 2 à 4, **caractérisé en ce que** la section d'extrémité distale (42) de l'élément tubulaire (4) présente au niveau d'un côté extérieur (43a) de la paroi (43) une dépression concave (44) dans laquelle pénètre au moins une partie de la section d'extrémité proximale (3b) de la tige intérieure (3).

6. Cathéter selon la revendication 5, **caractérisé en ce que** la dépression (44) est ménagée dans la paroi (43) par déformation de la paroi (43) de l'élément tubulaire (4).

7. Cathéter selon l'une des revendications 1, 4, 5 ou 6, **caractérisé en ce que** l'élément de raidissement (5) est serré dans la lumière (4a) de l'élément tubulaire (4) par la dépression (44).

8. Cathéter selon l'une des revendications 2 à 4, **caractérisé en ce que** la section d'extrémité proximale (3b) de la tige intérieure (3) est en prise avec l'élément tubulaire (4) **en ce que** la section d'extrémité proximale (3b) de la tige intérieure (3) pénètre dans la section d'extrémité distale (42) de l'élément tubulaire (4) et s'étend dans la lumière (4a) de l'élément tubulaire (4) à travers la section d'extrémité distale (42) de l'élément tubulaire (4).

9. Cathéter selon l'une des revendications 2 à 4 ou selon la revendication 8, **caractérisé en ce que** la paroi (43) de l'élément tubulaire (4) présente, dans une section de liaison (41) de l'élément tubulaire (4) qui relie la section d'extrémité proximale (40) de l'élément tubulaire (4) à la section d'extrémité distale (42) de l'élément tubulaire (4), un orifice de passage (4c) à travers lequel une partie de la section d'extrémité proximale (3b) de la tige intérieure (3) est guidée hors de la lumière (4a) de l'élément tubulaire (4).

10. Cathéter selon la revendication 9, **caractérisé en ce que** l'orifice de passage (4c) s'étend le long d'une direction (R) s'étendant perpendiculairement à un axe longitudinal (x) de la tige extérieure (2).

11. Cathéter selon l'une des revendications 2 à 4 ou selon l'une des revendications 8 à 10, **caractérisé en ce que** l'élément de raidissement (5) est calé dans la lumière (4a) de l'élément tubulaire (4) au sein de la section d'extrémité proximale (40) de l'élément tubulaire (4).

12. Cathéter selon l'une des revendications précédentes, **caractérisé en ce que** la section proximale de tige extérieure (20) présente une structure tubulaire métallique (201) qui est reliée à l'élément tubulaire (4) et/ou à un élément formant tuyau flexible (210) de la section distale de tige extérieure (21) par l'intermédiaire d'un élément formant tuyau flexible (200) de la section proximale de tige extérieure (20), l'élément de raidissement (5), en particulier une section d'extrémité proximale (50) de celui-ci, s'étendant jusque dans une section de la lumière (2a) qui est entourée par la structure tubulaire métallique (201).

13. Cathéter selon l'une des revendications précédentes, **caractérisé en ce que** la section distale de tige extérieure (21) présente un élément formant tuyau (210) qui est relié à l'élément tubulaire (4) et/ou à un élément formant tuyau flexible (200) de la section proximale de tige extérieure (20), l'élément de raidissement (5), en particulier une section d'extrémité distale (50) de celui-ci, s'étendant jusque dans une section de la lumière (2a) qui est entourée par l'élément formant tuyau (210) de la section distale de tige extérieure (21).

14. Procédé de fabrication d'un cathéter (1), en particulier selon l'une des revendications précédentes, dans lequel une section proximale de tige extérieure (20) et une section distale de tige extérieure (21) du cathéter (1) à fabriquer sont mises à disposition, dans lequel est agencée une tige intérieure (3) présentant une lumière pour fil de guidage (3a) permettant d'accueillir un fil de guidage (30), dans une section distale (21a), entourée par la section distale de tige extérieure (21), d'une lumière (2a) du cathéter (1) à fabriquer, et dans lequel un élément tubulaire métallique (4) est mis à disposition, dans lequel un élément de raidissement (5) métallique et allongé longitudinalement est fixé dans une lumière (4a) de l'élément tubulaire (4) par déformation de l'élément tubulaire (4), dans lequel une section d'extrémité proximale (3b) de la tige intérieure (3) est mise en prise avec l'élément tubulaire (4), et dans lequel la section proximale de tige extérieure (20) et la section distale de tige extérieure (21) sont reliées à l'élément tubulaire (4) de façon telle que l'élément de raidissement (5) est guidé depuis l'élément tubulaire (4) dans une section proximale (20a), entourée par la section proximale de tige extérieure (20), de la lumière (2a) et dans la section distale (21a) de la lumière (2a).
